(19)

Europäisches
Patentamt
European
Patent Office
Office européen
des brevets

(11) **EP 4 417 978 A1**

(12) **EUROPEAN PATENT APPLICATION**
published in accordance with Art. 153(4) EPC

(43) Date of publication:
**21.08.2024 Bulletin 2024/34**

(51) International Patent Classification (IPC):
***G01N 35/04*** *(2006.01)*    ***G01N 33/86*** *(2006.01)*

(21) Application number: **22880821.8**

(52) Cooperative Patent Classification (CPC):
**G01N 33/86; G01N 35/04**

(22) Date of filing: **30.09.2022**

(86) International application number:
**PCT/JP2022/036831**

(87) International publication number:
**WO 2023/063125 (20.04.2023 Gazette 2023/16)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **15.10.2021 JP 2021169648**

(71) Applicant: Sekisui Medical Co., Ltd.
**Tokyo 103-0027 (JP)**

(72) Inventors:
• **KAWABE, Toshiki**
**Tokyo 103-0027 (JP)**
• **OGASAWARA, Kosuke**
**Tokyo 103-0027 (JP)**
• **IIJIMA, Yumi**
**Tokyo 103-0027 (JP)**

(74) Representative: **Wächtershäuser & Hartz
Patentanwaltspartnerschaft mbB
Weinstraße 8
80333 München (DE)**

(54) **METHOD FOR ANALYZING BLOOD COAGULATION REACTION**

(57)    A method for analyzing a blood coagulation reaction comprises, by a blood coagulation analysis device, measuring a coagulation reaction of a sample solution including a subject blood specimen, determining whether or not the coagulation reaction of the sample solution is completed, and when it is determined that the coagulation reaction is completed, terminating measurement of the coagulation reaction of the sample solution. The blood coagulation analysis device includes a reaction table which is rotated in one direction, and the reaction table includes a measurement port to which a cell can be attached. The coagulation reaction of the sample solution in the cell supplied to the measurement port is measured, and time series data of the measured coagulation reaction is accumulated. Whether or not the coagulation reaction is completed is determined from the accumulated time series data of the coagulation reaction, and when it is determined that the coagulation reaction is completed, measurement of the coagulation reaction of the sample solution is terminated, and the cell containing the sample solution is removed from the measurement port.

Fig. 5

EP 4 417 978 A1

**Description**

Field of the Invention

The present invention relates to a method for analyzing blood coagulation reaction.

Background of the Invention

A blood coagulation test is a test for diagnosing the blood coagulation ability of a subject by examining the coagulation reaction of the subject's blood specimen. In the blood coagulation test, generally, after adding a predetermined reagent to a blood specimen, the coagulation reaction is measured over time, and the blood coagulation time is measured based on the time series data of the obtained coagulation reaction. The blood coagulation time is used as an indicator for detecting an anomaly in blood coagulation ability. In recent years, automatic analysis devices for measuring the blood coagulation time have been widely used.

When measuring the blood coagulation time with an automatic analysis device, a specimen and a reagent are put into a cell, and the coagulation reaction in the cell is measured over time with an optical method and the like. Since the measurement of the coagulation reaction is performed in a measurement port of the device, a cell is supplied to and removed from the measurement port for each measurement. A common automatic analysis device includes a plurality of measurement ports for measuring the coagulation reaction in a cell, thereby being able to measure the coagulation reaction in a plurality of cells in parallel. A specimen is dispensed into a cell supplied to the measurement port, and thereafter the coagulation reaction in the cell is measured after a reagent is added to the cell until the cell is removed from the measurement port. In normal automatic analysis devices, the measurement time is set in advance, and is the same between cells (specimens).

Patent Literatures 1 and 2 describe a blood coagulation analysis device comprising a drive mechanism which rotates and stops a table in a reciprocating direction, the table including a plurality of measurement unit modules for measuring the coagulation time of a cell, the blood coagulation analysis device capable of moving the measurement unit modules to an attachment/detachment position of a cell, a specimen dispensing position, and a reagent dispensing position, and stopping the measurement unit modules. Patent Literature 3 describes a blood coagulation analysis device comprising a fixed table including a plurality of light measurement ports for measuring the coagulation time of cuvette (cell), and a cell transfer arm which supplies a cell including a specimen to the light measurement ports on the table.

In conventional blood coagulation tests, it is common to measure the coagulation reaction for the time sufficient for the coagulation reaction to complete, and then to calculate the coagulation time based on the obtained data. However, an abnormal specimen which contains an anomaly in a blood coagulation reaction often shows a delay in the initiation and progress of the coagulation reaction, and as a result, it tends to take a long time for the coagulation reaction to complete. Therefore, when measuring the coagulation reaction of a large number of blood specimens with a conventionally common automatic analysis device, in order to prevent measurement termination midway through the coagulation reaction, it has been necessary to set a long measurement time in consideration of existence of an abnormal specimen with prolonged coagulation time. On the other hand, since most specimens to be measured are normal samples, the longer the measurement time is set, the lower the overall analysis efficiency becomes.

Citation List

Patent Literature

Patent Literature 1: JP-A-H06-109742
Patent Literature 2: JP-A-H06-109743
Patent Literature 3: JP-A-2000-321286

Summary of the Invention

Technical Problem

The present invention relates to appropriately adjusting the measurement time of each blood specimen in blood coagulation reaction analysis by an automatic analysis device, and improving the analysis efficiency.

Solution to Problem

The present invention provides the following.

[1] A method for analyzing a blood coagulation reaction, comprising,

by a blood coagulation analysis device, measuring a coagulation reaction of a sample solution including a subject blood specimen;
determining whether or not the coagulation reaction of the sample solution is completed; and
when it is determined that the coagulation reaction is completed, terminating measurement of the coagulation reaction of the sample solution,
wherein the blood coagulation analysis device includes a reaction table which is rotated in one direction,
the reaction table includes a measurement port to which a cell can be attached,
the coagulation reaction of the sample solution in the cell supplied to the measurement port is measured,
time series data of the measured coagulation reaction is accumulated,
whether or not the coagulation reaction is completed is determined from the accumulated time series data of the coagulation reaction, and
when it is determined that the coagulation reaction is completed, the measurement of the coagulation reaction of the sample solution is terminated, and the cell containing the sample solution is removed from the measurement port.

[2] The method according to [1], wherein the reaction table has a disk-like shape.
[3] The method according to [1] or [2], wherein the reaction table performs an intermittent rotation operation, and a time for which the reaction table completes one full round of rotation is from 320 to 380 seconds.
[4] The method according to [3], wherein an interval of the intermittent rotation operation is $\alpha$ seconds, and a number of the measurement port on the reaction table is $\beta$, where $\alpha$ = from 8 to 20, $\beta$ = from 16 to 47, and [$\alpha \times \beta$] = from 320 to 380 seconds, and
a rotation angle of the reaction table in one intermittent rotation operation is [$360°/\beta$].
[5] The method according to any one of [1] to [4], wherein the measurement of the coagulation reaction of the sample solution including the subject blood specimen comprises:

supplying a cell to the measurement port of the reaction table;
dispensing the subject blood specimen to the cell, and heating the subject blood specimen;
dispensing a reagent to the cell containing the subject blood specimen to prepare the sample solution; and
measuring the coagulation reaction of the sample solution, and the supplying of the cell to the measurement port, the dispensing of the subject blood specimen to the cell, and the dispensing of the reagent to the cell containing the subject blood specimen are performed when the measurement port reaches a predetermined position for each,
and
the terminating the measurement of the coagulation reaction of the sample solution is performed when the measurement port reaches a predetermined position for the terminating.

[6] The method according to [5], wherein when the measurement port of the reaction table reaches a predetermined position A, the cell is supplied to the measurement port, when the measurement port reaches a predetermined position B, the subject blood specimen is dispensed to the cell, when the measurement port reaches a predetermined position C or a predetermined position D, the reagent is dispensed to the cell, and thereafter, when the measurement port reaches the predetermined position A or a predetermined position E, the cell is removed from the measurement port.
[7] The method according to [6], wherein the position A is disposed on a location shifted from the position E in a rotation direction by one intermittent rotation operation of the reaction table.
[8] The method according to [6] or [7], wherein when the cell supplied to the measurement port reaches the position E on the reaction table, if it has been determined that the coagulation reaction of the sample solution in the cell is completed, measurement of the coagulation reaction of the sample solution is terminated, and then, at the position E, or when the cell reaches the position A, the cell is removed from the measurement port.
[9] The method according to any one of [6] to [8], wherein when the cell supplied to the measurement port reaches the position E on the reaction table, if it has not been determined that the coagulation reaction of the sample solution in the cell is completed, the cell is rotated on the reaction table without being removed from the measurement port, and measurement of the coagulation reaction of the sample solution in the cell is continued.

[10] The method according to any one of [6] to [9], wherein after dispensing the subject blood specimen to the cell supplied to the measurement port at the position B, when the measurement port reaches the position C or the position D for the first time, the reagent is dispensed to the cell.

[11] The method according to any one of [6] to [9], wherein after dispensing the subject blood specimen to the cell at the position B, before dispensing the reagent to the cell at the position C or the position D, the reaction table is rotated one or more times, during which heating of the subject blood specimen is continued.

Advantageous Effects of Invention

According to the present invention, the efficiency of analysis of the blood coagulation reaction by an automatic analysis device can be improved without complicating the configuration and operation of the device.

Brief Description of Drawings

[Figure 1] Figure 1 is a conceptual diagram of a system for blood coagulation analysis used in a method of the present invention.

[Figure 2] A: a diagram for describing the configuration of an analysis module 100. B: a diagram for describing the configuration of a measurement port 2.

[Figure 3] Figure 3 is a diagram for describing a rotation operation of a reaction table 1.

[Figure 4] Figure 4 illustrates steps from supplying of a cell to the measurement port 2 to removal of the cell after completion of measurement. A: a case of measurement by a one-reagent system, B: a case of measurement by a two-reagent system.

[Figure 5] Figure 5 illustrates a flow of coagulation reaction analysis with a method of the present invention using the analysis module 100.

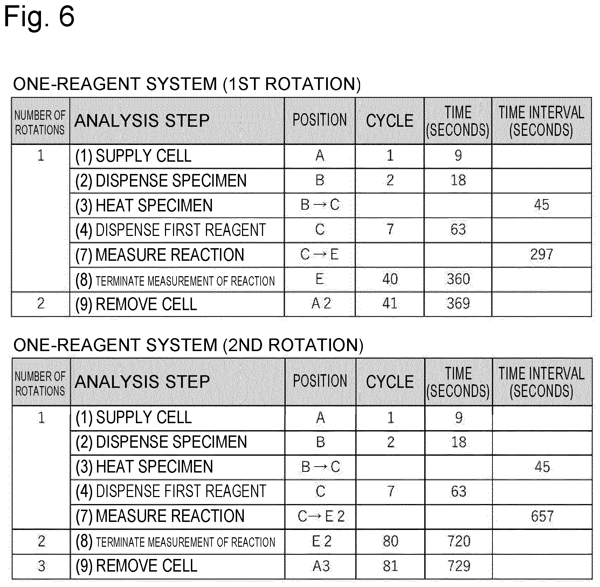

[Figure 6] Figure 6 illustrates a table which lists how many rotations of the reaction table 1 were made, as well as an analysis step at that time, a position or interval where the analysis step was performed, a cycle in which the analysis step was performed, the time until the analysis step was started since the analysis was started, and the time required for the analysis step (sample heating, sample solution heating, and reaction measurement). Upper table: a case where measurement was terminated when the reaction table 1 was rotated once, lower table: a case where measurement was terminated when the reaction table 1 was rotated twice. In a case of measurement by the one-reagent system.

[Figure 7] Figure 7 is the same as Figure 6. In a case of measurement by the two-reagent system. Upper table: a case where measurement was terminated when the reaction table 1 was rotated once, middle table: a case where measurement was terminated when the reaction table 1 was rotated twice, and lower table: a case where measurement was terminated when the reaction table 1 was rotated three times.

[Figure 8] Figure 8 is the same as Figure 6. In a case of a delayed reaction of a cross mixing test. Upper table: a case where measurement was terminated when the reaction table 1 was rotated three times, lower table: a case where measurement was terminated when the reaction table 1 was rotated four times.

[Figure 9] Figure 9 illustrates coagulation reaction curves and APTTs of a sample measured with the method of the present invention. A: a normal sample, B and C: abnormal samples. In A and B, measurement was terminated when the reaction table 1 was rotated once. In C, measurement was terminated when the reaction table 1 was rotated twice.

Description of Embodiments

In analysis of a blood coagulation reaction, a predetermined reagent is added to a blood specimen to prepare a sample solution, and the blood coagulation reaction thereof is measured. The blood coagulation reaction is generally represented by a coagulation reaction curve which indicates changes over time in the amount of coagulation reaction. In the present specification, a blood specimen, a blood coagulation reaction, and a blood coagulation time may be simply called a sample, a coagulation reaction, and a coagulation time, respectively.

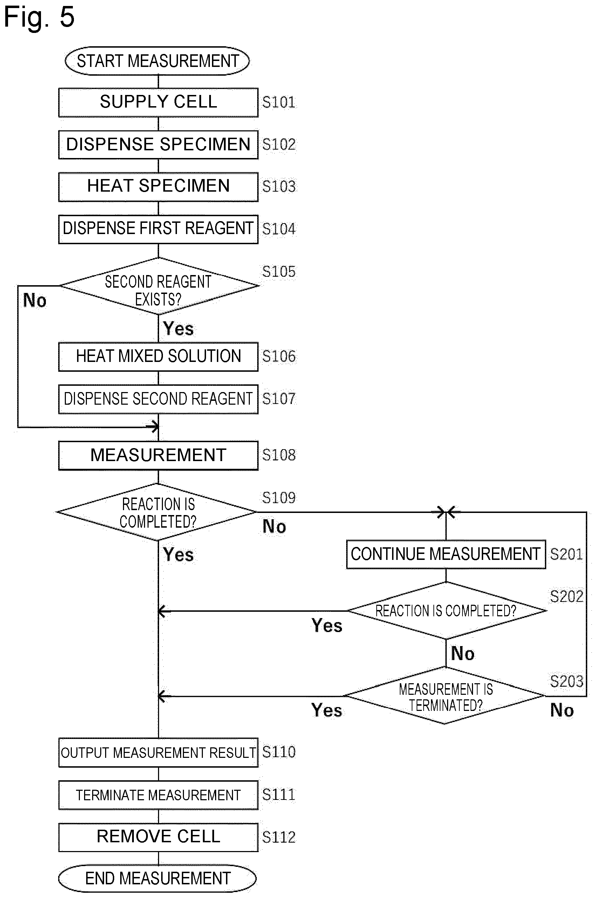

Conventional blood coagulation analysis devices typically measure the coagulation reactions of a large number of sample solutions in parallel. The measurement time of the coagulation reactions is set in advance, and is basically constant between the sample solutions. Therefore, in analysis by blood coagulation analysis devices, it is necessary to set a longer measurement time in consideration of the possibility that an abnormal sample with prolonged coagulation time exists in a sample group to be tested. Although the aforementioned Patent Literatures 1 and 2 indicate the possibility that the measurement time can be changed for each sample (for example, Figure 5 of Patent Literature 1), neither a specific technique nor a control mechanism for individually controlling the measuring time for each sample is disclosed.

Meanwhile, in a method for analyzing a blood coagulation reaction of the present invention (hereinafter also referred to as the method of the present invention), in parallel with the measurement of the coagulation reaction of a sample solution in a blood coagulation analysis device, it is determined whether or not the coagulation reaction of the sample solution is completed. When it is determined that the coagulation reaction is completed, the measurement of the coagulation reaction of the sample solution is terminated, and otherwise, the measurement is basically continued until the coagulation reaction is completed. Accordingly, in the method of the present invention, the measurement time of a coagulation reaction may be changed for each sample solution. The method of the present invention can optimize the measurement time of a coagulation reaction for each sample solution, thereby enabling reduction of overall measurement time, so to speak, improvement of analysis efficiency.

On the other hand, a blood coagulation analysis device generally performs complicated operations, such as supplying a cell to a measurement port, dispensing of a sample and a reagent to the cell, movement of the measurement port, and removal of the cell from the measurement port. However, complication of the operations of the device easily results in blurring and noise in measurement values, which in turn leads to a decrease in measurement accuracy. For example, in cases of the devices described in Patent Literatures 1 and 2, a measurement port is moved to a predetermined position by rotating a table on which the measurement port is mounted in a reciprocating direction. Although such reciprocating operations make it possible to immediately move a cell to a target position with the minimum moving distance, since the cell is swung and the sample solution in the cell is shaken with the reciprocating operations of the table, noise is mixed into a coagulation reaction curve (measurement data), which will have a significant negative influence on measurement values (coagulation times). It is desired to prevent blurring and noise in measurement values by simplifying the operations of the device.

The method for analyzing a blood coagulation reaction of the present invention comprises the following:

by a blood coagulation analysis device, measuring a coagulation reaction of a sample solution including a subject blood specimen;
determining whether or not the coagulation reaction of the sample solution is completed; and
when it is determined that the coagulation reaction is completed, terminating measurement of the coagulation reaction of the sample solution.

The blood coagulation analysis device used in the method of the present invention comprises a reaction table including a plurality of equally spaced measurement ports to which a cell can be attached. A cell into which a sample solution is to be put can be supplied to each one of the measurement ports. At the time of analysis, the coagulation reactions of the sample solution in the cells supplied to the measurement ports are measured. Although it is preferable that the coagulation reactions are optically measured, well-known methods may be used, and the methods are not particularly limited. In the blood coagulation analysis device used in the method of the present invention, each measurement port is moved to an attachment/detachment position of a cell and to a dispensing position of a sample and a reagent by rotating the reaction table in one direction. Such a mechanism simplifies the configuration and operation of the device, and reduces blurring and noise in measurement values due to the operation of the device.

The data of the measured coagulation reaction of the sample solution is successively sent to a data processing unit, and is accumulated as the time series data of the coagulation reaction. The data processing unit determines whether or not the coagulation reaction of the sample solution is completed from the time series data of the accumulated coagulation reaction. When it is determined that the coagulation reaction is completed, the measurement of the coagulation reaction of the sample solution is terminated. The cell containing the sample solution whose coagulation reaction is completed is removed from the measurement port, and is discarded.

Hereinafter, referring to the drawings, an embodiment of a method for analyzing a blood coagulation reaction according to the present invention will be described. However, the embodiment of the present invention is not limited to the following description. Additionally, each embodiment can be combined with other embodiments or conventional technologies.

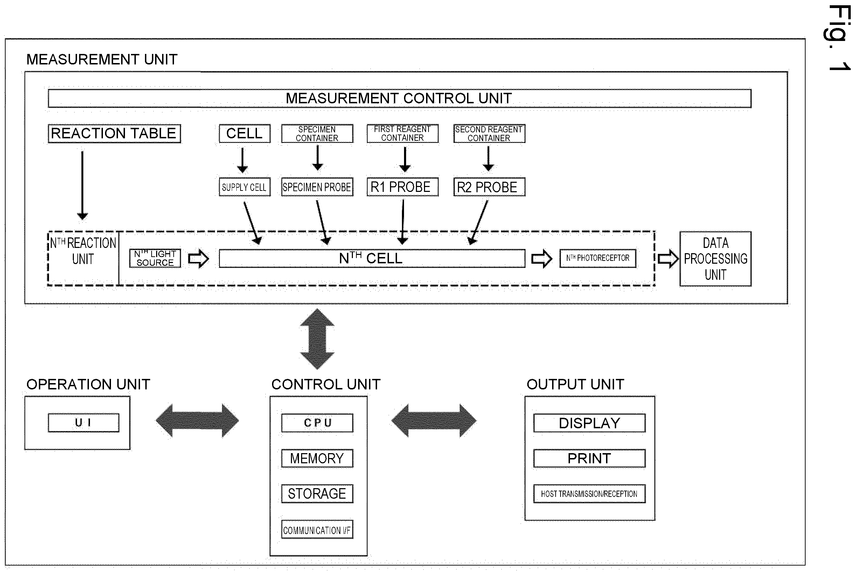

Figure 1 illustrates a conceptual diagram of an embodiment of a system for blood coagulation analysis used in the method of the present invention. The system illustrated in Figure 1 includes a measurement unit, a control unit, an operation unit, and an output unit for performing blood coagulation analysis. The blood coagulation analysis by the present system may be performed by a program for performing the method of the present invention.

The control unit controls the operation of the entire system. The control unit may be constituted by, for example, one or more computers. The control unit includes a CPU, a memory, a storage, a communication interface (I/F), and the like, and performs processing of a command from the operation unit, control of the operation of the measurement unit, saving of measurement data and analysis results received from the measurement unit, control of output of the measurement data and the analysis results by the output unit, and the like. Further, the control unit may be connected to other equipment, such as an external media and a host computer. The control of the system by the control unit may be performed by a program.

The operation unit obtains an input from an operator, and transmits the obtained input to the control unit. For example, the operation unit includes a user interface (UI), such as a keyboard and a touch panel. The output unit outputs measurement data and analysis results of the measurement unit under control of the control unit.

The measurement unit is mainly constituted by an analysis module 100, which will be described later, and obtains measurement data of a coagulation reaction of a sample solution including a subject specimen. In the measurement unit, n subject specimens are analyzed in parallel in n reaction units (for example, a measurement port 2, which will be described later). The obtained measurement data is analyzed in the data processing unit, and it is determined whether or not the coagulation reaction is completed. The data processing unit can also perform arithmetic processing of coagulation time calculation and the like. Measurement and analysis of data are controlled by the measurement control unit. Alternatively, measurement and analysis of data may be controlled by the control unit via the measurement control unit.

In the embodiment illustrated in Figure 1, the measurement control unit and the data processing unit of the measurement unit are included in the measurement unit. In another embodiment, the measurement control unit and the data processing unit are included in the control unit. In still another embodiment, the measurement control unit and the data processing unit constitute separate units. In yet another embodiment, the measurement control unit and the data processing unit are integrated and included in the measurement unit or the control unit.

An analysis result in the data processing unit is sent to the output unit under control of the control unit. An output from the output unit can take any forms, such as display on a screen, transmission to a host computer, and printing. Output information from the output unit can include the data of a coagulation reaction curve, a coagulation time, and the like.

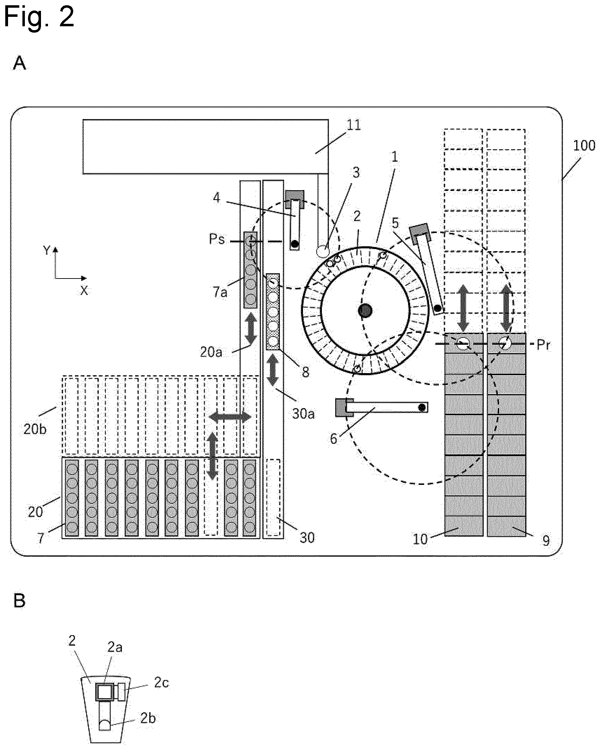

Figure 2A illustrates the configuration of the analysis module 100, which is an embodiment of the blood coagulation analysis device used in the method of the present invention. Note that, in the following description of Figure 2A, a description will be given by assuming that a horizontal direction of the figure is an X-axis, a vertical direction is a Y-axis, and a direction perpendicular to these is a Z-axis.

The analysis module 100 includes a reaction table 1, the measurement port 2, a cell transfer unit 3, a sample dispensing unit 4, a first reagent dispensing unit 5, and a second reagent dispensing unit 6. Further, in the analysis module 100 illustrated in Figure 2A, sample racks (7 and 7a), a diluent rack 8, a first reagent installation unit 9, a second reagent installation unit 10, a cell housing unit 11, a sample installation unit 20, a sample rack Y-axis moving lane 20a, a sample rack X-axis moving lane 20b, a diluent rack installation unit 30, and a diluent rack moving lane 30a are illustrated.

Additionally, although not illustrated in Figure 2A, the analysis module 100 includes a drive mechanism, such as a motor, a pump, and an actuator, for driving the reaction table 1, the cell transfer unit 3, the sample dispensing unit 4, the reagent dispensing units 5 and 6, and the like, and for moving the sample racks and the diluent rack.

The analysis module 100 may also include a control unit 12 for controlling the operation of the drive mechanism, the measurement by the measurement port 2, or the analysis of measurement data. The analysis module 100 may also include a data processing unit 13 for analyzing the measured coagulation reaction data, determining whether or not the coagulation reaction of the sample solution is completed, and calculating the coagulation time of a subject specimen. Alternatively, the control unit 12 and the data processing unit 13 may be installed separately from the analysis module 100.

Although the reaction table 1 illustrated in Figure 2A is a disk-like table, the reaction table 1 may be constituted by other structures that can perform a rotation operation, for example, a rotating conveyor belt. Accordingly, although it is preferable that the orbit of the reaction table 1 is a circle (a perfect circle, an elliptical shape), and a perfect circle is more preferable, the orbit may be a shape other than these. The reaction table 1 includes a plurality of measurement ports 2. Preferably, the measurement ports 2 in Figure 2A are arranged along the circumference of the reaction table 1. Accordingly, the measurement ports 2 are rotated along the orbit of the reaction table 1. Note that, in the present specification, "the rotation of the measurement port 2" and "the rotation of a cell" refer to the rotation of the measurement port 2 on the reaction table 1 or a cell attached thereto along the orbit of the reaction table 1, together with the rotation of the reaction table 1.

In order to simplify the operation control of the reaction table 1, it is preferable that the plurality of measurement ports 2 are installed at equal intervals on the reaction table 1. The reaction table 1 is rotated in one direction by the drive mechanism (not illustrated). In consideration of accessing to the measurement port 2 from the cell transfer unit 3, the sample dispensing unit 4, and the reagent dispensing units 5 and 6 and the operations of these units, it is preferable that the reaction table 1 intermittently performs rotation operations. The number of the measurement ports 2 installed in the reaction table 1 may be appropriately adjusted depending on the size of the reaction table 1, the cycle of the intermittent rotation operation, the time required for one rotation, and the like.

As illustrated in Figure 2B, each of the measurement ports 2 includes a cell attachment portion 2a. A cell is supplied to the cell attachment portion 2a by the cell transfer unit 3 from the cell housing unit 11. The cell transfer unit 3 also serves for removing the cell whose measurement is completed from the cell attachment portion 2a. It is preferable that a heating function of heating a subject specimen and a sample solution which are dispensed to a cell is provided around the cell attachment portion 2a in the measurement port 2.

The measurement port 2 includes a detection unit for measuring the coagulation reaction of the sample solution in the cell. The detection unit is preferably a mechanism for optically measuring the coagulation reaction of the sample solution. In the embodiment illustrated in Figure 2B, the detection unit includes a light source 2b which emits light to the sample solution in the cell, and a light measurement unit 2c for measuring scattered light generated when the light incident on the sample solution is scattered 90 degrees to the side. Alternatively, the transmission light from the sample solution or the absorbance of the sample solution may be measured in the light measurement unit 2c. In a preferred embodiment, the light source 2b includes a LED, and the light measurement unit 2c includes a photodiode.

The detection unit sequentially obtains, as measurement data, the photometric quantity from the sample solution at preset time intervals. The time interval for obtaining data is preferably K/10 seconds (K = an integer of from 1 to 5), and is more preferably 0.1 seconds.

The cell transfer unit 3, the sample dispensing unit 4, the first reagent dispensing unit 5, and the second reagent dispensing unit 6 are arranged around the reaction table 1. The cell transfer unit 3 serves to supply a cell to the cell attachment portion 2a of the measurement port 2 from the cell housing unit 11, or to remove the cell from the cell attachment portion 2a. The sample dispensing unit 4 serves to dispense a subject specimen from the sample rack 7a and a diluent from the diluent rack 8 to the cell in the cell attachment portion 2a. The first reagent dispensing unit 5 and the second reagent dispensing unit 6 serve to dispense a first reagent of the first reagent installation unit 9 and a second reagent of the second reagent installation unit 10 to the cell in the cell attachment portion 2a, respectively.

In the analysis module 100 illustrated in Figure 2A, supplying of a cell to and removing of the cell from the measurement port 2 are both performed by the cell transfer unit 3. In another embodiment, instead of one cell transfer unit 3, a cell supplying unit 3a and a cell removing unit 3b perform supplying of a cell to and removing of the cell from the measurement port 2, respectively.

In Figure 2A, the sample dispensing unit 4, the first reagent dispensing unit 5, and the second reagent dispensing unit 6 are rotating arms which are rotated around the positions indicated by black dot marks, and the circular broken lines around the black dot marks indicate the movable routes (trajectories) of the arms. The tip of each of the rotating arms includes a dispensing probe.

A cell supplied to the measurement port 2 is preferably a disposable cell. Preferably, the cells in the cell housing unit 11 are aligned by an alignment supplying unit (not illustrated), and thereafter supplied to the cell transfer unit 3. The cell transfer unit 3 supplies a cell to the cell attachment portion 2a. The cell after measurement is removed from the cell attachment portion 2a by the cell transfer unit 3, and is discarded to a cell discarding unit (not illustrated). Preferably, the cell transfer unit 3 includes a cell detection sensor (not illustrated) for detecting whether or not a cell is in the cell attachment portion 2a.

The sample rack 7 houses sample containers (blood collection tubes, sample cups, and the like) containing subject specimens. The sample rack 7 is arranged in the sample installation unit 20. In Figure 2A, the sample rack 7 can store a maximum of five sample containers at the same time, and positions from 1 to 5 are sequentially assigned to the housing locations of the sample containers from the top toward the end in a Y axial direction (from an upper side to a lower side in the figure). Additionally, ten sample racks 7 can be arranged in the sample installation unit 20. The sample rack 7 is moved in the directions of arrows from the sample installation unit 20, and is transferred to the sample rack Y-axis moving lane 20a through the sample rack X-axis moving lane 20b. The subject specimens arranged in the sample rack 7a which has been moved to the sample rack Y-axis moving lane 20a are sucked by the sample dispensing unit 4 at a predetermined position (Ps). When suction of the samples from all the sample containers of the sample rack 7a has been performed, the sample rack 7a is returned to the original position of the sample installation unit 20 through the reverse route. Then, another sample rack 7 which is set to the sample installation unit 20 is transferred to the sample rack Y-axis moving lane 20a.

The diluent rack 8 houses containers containing sample diluent used for dilution of the subject specimens. For example, the diluent rack 8 houses the containers containing normal plasma used for a cross mixing test. The sample rack 8 can house a maximum of five containers at the same time, and positions from 1 to 5 are sequentially assigned to the housing locations of the containers from the top toward the end in the Y axial direction (from the upper side to the lower side in the figure). In Figure 2A, the diluent rack 8 is arranged in the diluent rack installation unit 30, and can move on the diluent rack installation unit 30 in the Y axial direction (the directions of the arrows). With movement of the diluent rack 8, each container containing a sample diluent and housed in the rack 8 is moved to a predetermined position, and the sample diluent in the container is sucked by the sample dispensing unit 4.

The reagents used for preparation of the sample solution is housed in the first reagent installation unit 9 and the second reagent installation unit 10. In Figure 2A, the first reagent installation unit 9 and the second reagent installation unit 10 can house ten containers containing the first reagent and ten containers containing the second reagent, respectively (gray portions). In order to distinguish the installation positions of the reagent containers, position numbers from 1 to 10 are given from the top toward the end in the Y axial direction (from the upper side to the lower side in the figure). The first reagent installation unit 9 and the second reagent installation unit 10 are movable in the Y axial direction (the directions of the arrows), and the movable ranges for these are indicated by dotted lines.

The first reagent and the second reagent are used in accordance with test items. For example, in the case of measurement of prothrombin time (PT), only the first reagent is used, and in the case of measurement of activated partial thromboplastin time (APTT), the first reagent and the second reagent are used. Preferably, the first reagent and the second reagent which are used for analysis of the same sample are arranged in the respective reagent installation units so as to be arranged side by side. In this case, when only the first reagent is used for analysis, the adjacent position for the second reagent installation unit 10 is empty. The first reagent and the second reagent which are used for analysis are conveyed to a predetermined position (Pr) by movement of the first reagent installation unit 9 and the second reagent installation unit 10, and are sucked by the first reagent dispensing unit 5 and the second reagent dispensing unit 6, respectively.

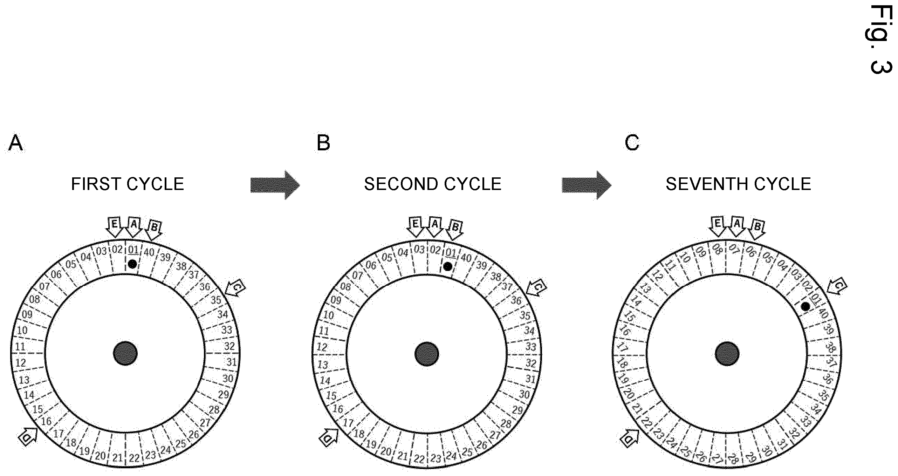

Next, referring to Figure 2A and Figure 3, the operation of the reaction table 1 will be described.

In Figure 2A, 40 measurement ports 2 are arranged at equal intervals on the circumference of the disk-like reaction table 1. The cell transfer unit 3, the sample dispensing unit 4, the first reagent dispensing unit 5, and the second reagent dispensing unit 6 are arranged around the reaction table 1, and each of these accesses the reaction table 1 at one specific place. The access positions to the reaction table 1 of the cell transfer unit 3, the sample dispensing unit 4, the first reagent dispensing unit 5, and the second reagent dispensing unit 6 are called a position A, a position B, a position C, and a position D, respectively. The position A, the position B, the position C, and the position D are set in this order along the rotation direction of the reaction table 1. That is, there are the position A, the position B, the position C, and the position D in this order from upstream in the rotation direction.

That is, supplying and removing of a cell to and from the measurement port 2 on the reaction table 1 by the cell transfer unit 3 are performed when the measurement port 2 reaches the position A. When the reaction table 1 is rotated from there, and the measurement port 2 to which the cell has been supplied reaches the position B, a subject specimen is dispensed to the cell by the sample dispensing unit 4. When the reaction table 1 is further rotated, and the measurement port 2 reaches the position C, the first reagent is dispensed to the cell by the first reagent dispensing unit 5. When the reaction table 1 is further rotated, and the measurement port 2 reaches the position D, when required, the second reagent is dispensed to the cell by the second reagent dispensing unit 6. When the required reagent has been dispensed to the cell, coagulation reaction measurement is started. When setting a longer heating time for the subject specimen, after the subject specimen is dispensed to the cell at the position B, the first reagent may be dispensed to the cell at the position D, and then, coagulation reaction measurement may be started. Alternatively, when setting a further longer heating time for the subject specimen, the cell to which the subject specimen has been dispensed at the position B can be passed through the positions C and D without dispensing the reagents. In this case, after dispensing the subject specimen to the cell, before dispensing the reagent to the cell, the reaction table 1 is rotated once, twice, or three or more times, during which the heating of the subject specimen is continued. When the cell reaches the position C or the position D for the second time, the third time, or the fourth or more time, the first reagent and, if necessary, the second reagent are dispensed to the cell, and then, coagulation reaction measurement is started. The reaction table 1 proceeds toward a measurement termination determination point (the position E, which will be described later) while being rotated. In a case where measurement is terminated at the position E, when the cell in the measurement port 2 is returned to the position A, the cell in the measurement port 2 is removed from the measurement port 2 by the cell transfer unit 3. While the position E is located downstream of the position D in the rotation direction of the reaction table 1, the position E is located upstream of the rotation direction of the reaction table 1 with respect to the position A. Preferably, the position obtained by shifting the position E by one cycle of the reaction table 1 in the rotation direction is the position A. That is, when the position A is the most upstream in the rotation direction of the reaction table 1, the position E is arranged at the most downstream (see Figure 3).

In an embodiment, supplying and removing of the cell to and from the measurement port 2 are both performed at the position A. In another embodiment, supplying and removing of the cell to and from the measurement port 2 are performed at another position on the reaction table 1. For example, supplying of a cell may be performed at the position A, and removing of the cell may be performed at the position E after completion of measurement.

The rotation speed (or the time required for one rotation) of the reaction table 1 is adjusted so as to be able to secure the time for, during one rotation, the attachment and detachment of the cell to and from the measurement port 2, dispensing of the subject specimen and the reagent to the cell, heating of the subject specimen and the sample solution, and coagulation reaction measurement of the sample solution. That is, while the reaction table 1 is rotated once, a new cell is supplied to the measurement port 2 after removing the measured cell, if necessary, a subject specimen and the reagent are dispensed to the cell and heated for a predetermined period of time, and the coagulation reaction is measured for a predetermined period of time.

In a preferred embodiment, the heating time of sample is from 40 to 60 seconds, the heating time of sample solution after addition of the reagent is from 160 to 180 seconds, and the measurement time of coagulation reaction is from 120 to 140 seconds. Accordingly, from 320 to 380 seconds, which is obtained by adding these, is the time required for analysis of one sample. When the interval of the intermittent rotation operation of the reaction table 1 is $\alpha$ (seconds), and the number of the measurement ports 2 on the reaction table 1 is $\beta$, the time for the reaction table 1 to be rotated once is [α × β](seconds). Additionally, when the reaction table 1 has a disk-like shape, the rotation angle of the reaction table 1 in one intermittent rotation operation (one cycle) is [360°/β]. Appropriate ranges of α and β can be determined in consideration of the time required for one rotation, and the time required for one cycle of the reaction table 1 for performing predetermined analysis.

In a preferred embodiment, α = from 8 to 20, and β = from 16 to 47. However, the time [α × β] for the reaction table 1 to be rotated once is from 320 to 380 seconds.

In a more preferred embodiment, α = 8 and β = from 40 to 47. In another more preferable embodiment, α = 9 and β = from 36 to 42. In another more preferable embodiment, α = 10 and β = from 32 to 38. In another more preferable embodiment, α = 12 and β = from 27 to 31. In another more preferable embodiment, α = 15 and β = from 22 to 25. In another more preferable embodiment, α = 18 and β = from 18 to 21. In another more preferable embodiment, α = 20 and β = from 16 to 19.

For example, in the case of the disk-like reaction table 1 including the 40 measurement ports 2 illustrated in Figure 2A, it is intermittently rotated every 9 seconds by 9° (360°/40) for each cycle in the clockwise direction. The reaction table 1 is rotated once in 40 cycles (360 seconds), and each measurement port 2 is returned to the original position at the 41st cycle.

As illustrated in Figure 3, position signs from P01 to P40 can be assigned to the 40 measurement ports 2 on the reaction table 1, respectively. Referring to Figure 3, the rotation operation of the reaction table 1 will be described by taking the case of analyzing a subject specimen in P01 as an example. Figure 3A represents the reaction table 1 at the time of starting analysis (a first cycle), and P01 is at the position A at this time. When analysis is started, the reaction table 1 is rotated clockwise by a rotation angle of 9°, and 40 moves (40 cycles) constitute one rotation. Although due to arrangement with surrounding analysis processing units the reaction table 1 in Figure 2A is rotated clockwise during analysis, the reaction table 1 can be rotated counterclockwise during analysis, depending on the arrangement of the surrounding analysis processing units. However, since the reaction table 1 during analysis is rotated in one direction, the reaction table 1 in Figure 2A will not be rotated counterclockwise during analysis. Figure 3B represents the reaction table 1 in a second cycle, and P01 is at the position B at this time. Figure 3C represents the reaction table 1 in a seventh cycle, and P01 is at the position C at this time.

As an example, a case will be described where the APTT of a subject specimen in P01 is measured by the analysis module 100 in Figure 2A. At the position A, a new cell is supplied to P01. When there is already a cell in P01, an operation of removing the cell is performed first. The subject specimen, the first reagent, and the second reagent are dispensed to P01 at the positions from B to D. Measurement of a coagulation reaction is started after dispensing of the second reagent at the position D. When the reaction table 1 is rotated once, P01 is at the position E, and when the coagulation reaction of the sample in P01 has been completed at this time, the measurement of the coagulation reaction is terminated. In a case where the coagulation reaction of the sample in P01 has not been completed when the reaction table 1 is rotated once and P01 reaches the position E, P01 is moved to the position A again, and enters the next rotation, while the measurement of the coagulation reaction is continued.

By repeating the supplying and removing of the cell to and from the cell attachment portion 2a, wear materials originating from the cell and the cell attachment portion 2a may be accumulated on the reaction table 1 (particularly, around the attachment portion 2a). In such a case, it is assumed that there is a risk that wear materials are mixed into the cell or enter between the cell attachment portion 2a and a cell outer surface, thereby negatively influencing photometric data. It is considered that such a risk becomes higher when the number of times of using the cell attachment portion 2a becomes larger. As an example of the countermeasure for reducing this risk, a method is considered that controls the operation of the reaction table 1 so that the number of times of using each measurement port 2 is made equal. For example, the measurement port 2 which is different from the measurement port 2 used in the last analysis may be used for the next analysis. Specifically, the measurement port 2 next to the measurement port 2 used in the last analysis can be used for the next analysis. Taking Figure 3 for an example, when P01 has been used for the last analysis, P40 may be used for the next analysis, and P40 may be arranged at the position A when starting the analysis in the first cycle.

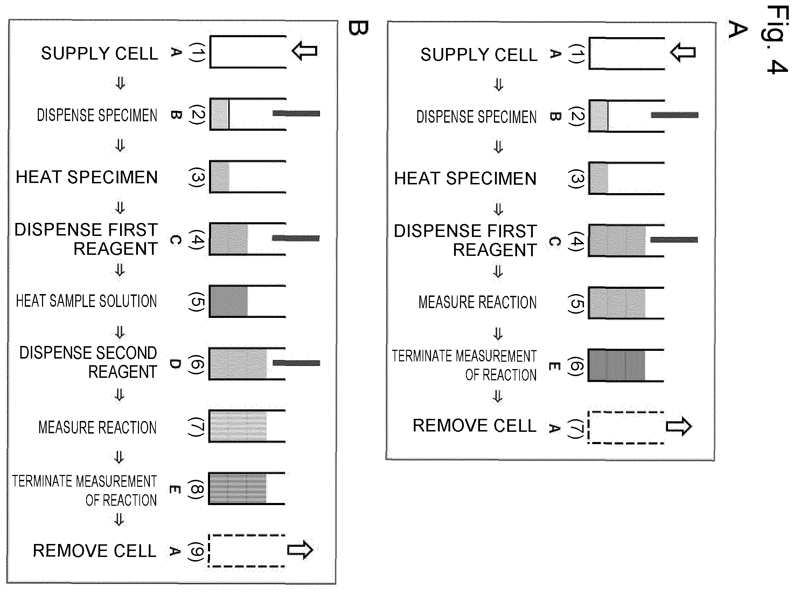

A series of steps from the supplying of a cell to the measurement port 2 on the reaction table 1 to the removing of the cell after termination of measurement are illustrated in Figures 4A and 4B. Both in Figures 4A and 4B, a first stage illustrates the states of a cell in the measurement port 2, a second stage indicates operation numbers, a third stage indicates the positions of the cell, and a fourth stage indicates analysis steps. In the case of measurement (for example, PT measurement) by a one-reagent system, after the first reagent is dispensed to the cell at the position C, the coagulation reaction of a sample solution is measured (A in Figure 4). In the case of measurement (for example, APTT measurement) by a two-reagent system, after the second reagent is dispensed to the cell at the position D, the coagulation reaction of the sample solution is measured (B in Figure 4). The data of the coagulation reaction measured by the light measurement unit 2c is successively sent to the data processing unit 13, and is accumulated as time series data. The data processing unit 13 determines whether or not the coagulation reaction of the sample solution is completed from the time series data of the coagulation reaction of the accumulated sample solution. When the coagulation reaction is completed at the time when the cell reaches the position E, the measurement is terminated, and the cell will be removed from the measurement port 2 at the next position A.

In a preferred embodiment, in the data processing unit 13, a coagulation reaction curve is created from the time series data of the accumulated coagulation reaction. If necessary, the coagulation reaction curve may be differentiated to create the speed data or acceleration data of the coagulation reaction. These coagulation reaction curve, speed data and acceleration data thereof can be used for determination of termination of a coagulation reaction.

Arbitrary methods can be used for determination of termination of a coagulation reaction. For example, a coagulation reaction termination point Re may be determined in accordance with any criteria, such as a point of time at which a coagulation reaction curve R reaches a plateau, a point of time at which a first-order differential curve of R (the speed data of a coagulation reaction) is decreased to 0 or a constant value after reaching a peak (the maximum speed) (refer to JP-2020-068877), and the earliest point at which the ratio of the integrated value of R in a minute time period becomes less than a threshold value (WO 2021/132552). The detection of Re in the data processing unit 13 is performed in parallel with the measurement of coagulation reaction data (in real time, so to speak).

As an embodiment of the method of the present invention, an example of the detection procedure of Re based on the method described in WO 2021/132552 will be described in detail. In a coagulation reaction curve R(i) (i is a measuring point number), the ratio of the integrated value of R(i) in a minute time period is specified as an integration ratio Z(i), and is calculated by the following equation:

$$Z(i)=\{(R(i+1)+R(i+2)+...+R(i+m)\}/(R(i-m)+R(i-m+1)+...+R(i-1)\}$$

In the above equation, i represents the measuring point number, and m can be appropriately set in accordance with the measurement conditions, analysis items, and the like of a coagulation reaction, and is m = from 10 to 30, for example. R(i) at the earliest measuring point or point of time at which Z(i) becomes less than a threshold value Zs is detected as the coagulation reaction termination point Re. Although Zs may be appropriately set in accordance with an analysis item, Zs is greater than 1 and is 1.100 or less, and for example, in the case of APTT measurement, Zs is preferably 1.050 or less, and more preferably, Zs is in the range from 1.010 to 1.001. In order to prevent erroneous detection of Re due to an initial reaction abnormality and the like, it is preferable that the calculation of Z(i) is performed after i reaches a predetermined calculation starting point, and after R(i) becomes a predetermined value or more. In the present procedure, while measuring a coagulation reaction, R(i) is obtained and Z(i) is calculated for in parallel, and Re is detected.

When Re is detected, and it is determined that the coagulation reaction of the sample solution is completed, a signal of termination of reaction is sent from the data processing unit 13 to the control unit 12. When the reaction table 1 is rotated once and P01 reaches the position E, if where the control unit 12 has received the signal of termination of reaction, the control unit 12 terminates the measurement of the coagulation reaction of the sample solution in P01. In that case, the cell in P01 is removed from the measurement port 2 at the position A in the next cycle.

When the reaction table 1 is rotated once, and P01 reaches the position E for the first time, if Re has not been detected, the measurement of the coagulation reaction of the sample solution in P01 is continued. The cell in P01 is not removed from the measurement port 2, and the cell in P01 enters a second rotation on the reaction table 1 while the measurement is continued.

When the reaction table 1 is rotated twice, and P01 reaches the position E for the second time, if the control unit 12 has received the signal of termination of reaction, the measurement of the coagulation reaction of the sample solution in P01 is terminated, and the cell in P01 is removed from the measurement port 2 at the position A in the next cycle. In a case where Re is not detected even when the reaction table 1 is rotated twice, the cell in P01 enters a third rotation on the reaction table 1 to continue being measured. The cell can be left and rotated on the reaction table 1 in a similar procedure until the termination of reaction is detected, so as to continue the measurement of the coagulation reaction.

In this manner, the measurement of the cell attached to the measurement port 2 and containing the sample solution can be continued until the coagulation reaction is completed.

In some abnormal samples, Re cannot be detected since the amount of change in the measurement data due to a coagulation reaction is small. Based on the existence of such abnormal samples, an upper limit threshold value for the measurement time of a cell containing a sample solution may be set in advance. When the cell in P01 is rotated several times on the reaction table 1, and the measurement time reaches the upper limit threshold value, if the control unit 12 has not received the signal of termination of reaction, the control unit 12 can cause the cell in P01 to be removed from the measurement port 2, and can send a result that there is no signal of termination of reaction to the data processing unit 13. The upper limit threshold value for the measurement time can be appropriately set in accordance with the measuring method (for example, the PT measurement, the APTT measurement, the cross mixing test) of coagulation time.

The plurality of measurement ports 2 arranged on the circumference of the reaction table 1 sequentially arrive the positions from A to E with the rotations of the reaction table 1, and if cells are supplied there, the analysis step of the method of the present invention will proceed. Accordingly, the analysis module 100 can sequentially and continuously perform analysis of the coagulation reactions of a plurality of sample solutions. Although the analysis by the analysis module 100 can be performed in one or more measurement ports 2, it is not necessary to perform analysis in all of the plurality of measurement ports 2.

As an embodiment, a flow of the coagulation reaction analysis by the method of the present invention using the analysis module 100 illustrated in Figure 2A is illustrated in Figure 5. The detailed procedure of the flow in Figure 5 is shown below.

S101: supply a cell to the measurement port 2 at the position A (a first cycle).

S102: dispense a sample to the cell at the position B (a second cycle).

S103: heat the sample up to a sixth cycle.

S104: dispense the first reagent to the cell at the position C (a seventh cycle).

S105: when there is the second reagent, proceed to S106, otherwise proceed to S108.

S106: heat the sample solution in which the first reagent has been added to the sample until a 25th cycle.

S107: dispense the second reagent to the cell at the position D (a 26th cycle).

S108: <Case of one-reagent system (the previous step is S105)>

Start reaction measurement (light measurement) after dispensing the first reagent to the cell in S104.

<Case of two-reagent system (the previous step is S107)>

Start reaction measurement (light measurement) after dispensing the first reagent to the cell in S107.

S109: continuously monitor whether or not the coagulation reaction is completed based on the obtained measurement data, and when the reaction is completed, proceed to S110.

When the cell arrives at the position E with the reaction not being completed, proceed to S201.

S110: when the reaction is completed, calculate the coagulation time, and output the calculation result. When the reaction is not completed, the content (*) in accordance with the analysis result of measurement data is output.

*: example) a flag indicating "no reaction" or "in the middle of a reaction", "the coagulation time > X seconds", and the like

Sill: terminate the measurement at the position E (a $40 \times$ Nth cycle, $N \geq 1$) .

S112: remove the cell from the measurement port 2 at the position A (a $40 \times$ N+1th cycle, $N \geq 1$), and end the measurement.

S201: continue the measurement, and the cell enters the next rotation.

(the $40 \times$ N+1th cycle)

S202: continuously monitor whether or not the coagulation reaction is completed based on the obtained measurement data, and when the reaction is terminated, proceed to S110.

When the cell reaches the position E with the reaction not being terminated, proceed to S203.

S203: Determine whether or not the cell enters the next rotation based on a setting condition, and when the measurement is terminated, proceed to S110.

When the measurement is continued, proceed to S201.

Operation examples of the analysis module 100 illustrated in Figure 2A in the coagulation reaction analysis with the method of the present invention will be shown below.

<Operation Example 1> PT and APTT measurement Analysis Conditions)

Sample: a sample at the position 1 of the sample rack 7a

Analysis item: Two items, i.e., PT and APTT (hereinafter, AP) (it is assumed that the measured value of PT is the coagulation time.)

Reagent: PT reagent for P01 (one-reagent system), APTT reagent for P02 (two-reagent system).

Measurement time: until the reaction table 1 is rotated once, for both PT and AP

Analysis Step)

(The First Cycle)

PT: the cell transfer unit 3 supplies a new cell to the cell attachment portion 2a of P01 of the measurement port 2 at the position A.

(The Second Cycle)

PT: 50 μL of a sample sucked from the sample container housed in the sample rack 7 is dispensed to the cell in P01 at the position B. The dispensed sample is heated on the measurement port 2.
AP: the cell transfer unit 3 supplies a new cell to the cell attachment portion 2a of P02 of the measurement port 2 at the position A.

(The Third Cycle)

AP: 50 μL of the sample sucked from the sample container housed in the sample rack 7 is dispensed to the cell in P02 at the position B. The dispensed sample is heated on the measurement port 2.

(The Seventh Cycle)

PT: at the position C, the first reagent dispensing unit 5 dispenses, to the cell in P01, 100 μL of a predetermined amount of the PT reagent sucked from a first reagent container installed in the first reagent installation unit 9 to prepare a sample solution. The obtaining of measurement data (reaction measurement) is started immediately after the reagent is dispensed.

(The Eighth Cycle)

AP: at the position C, the first reagent dispensing unit 5 dispenses, to the cell in P02, 50 μL of a predetermined amount of the APTT reagent sucked from a first reagent container installed in the first reagent installation unit 9. The dispensed reagent is heated on the measurement port 2 together with the dispensed sample.

(The 27th Cycle)

AP: at the position D, the second reagent dispensing unit 6 dispenses, to the cell in P02, 50 μL of a predetermined amount of calcium chloride liquid sucked from a second reagent container installed in the second reagent installation unit 10 to prepare a sample solution. The obtaining of measurement data (reaction measurement) is started immediately after the reagent is dispensed.

(The Mth Cycle (7 < M < 40))

PT: The coagulation reaction progress status of PT is analyzed in real time based on the obtained time series measurement data, and when Re (the coagulation reaction termination point) is detected, the coagulation time is calculated, and the calculated coagulation time is output as the PT value.

(The Nth Cycle (27 < N < 40))

AP: The coagulation reaction progress status of APTT is analyzed in real time based on the obtained time series measurement data, and when Re is detected, the coagulation time is calculated, and the calculated coagulation time is output as the APTT value.

(The 40th Cycle)

PT: Since it is determined that the coagulation reaction is completed (Re is detected) when the position E is reached, the reaction measurement of P01 is terminated, and the obtained measurement data is saved.

(The 41st Cycle)

PT: the cell in P01 which has reached the position A is removed and discard.
AP: Since it is determined that the coagulation reaction is completed (Re is detected) when the position E is reached, the reaction measurement of P02 is terminated, and the obtained measurement data is saved.

(The 42nd Cycle)

AP: the cell in P02 which has reached the position A is removed and discard.

<Operation Example 2> APTT measurement (coagulation time extended sample)

Analysis Conditions)

Sample: sample at the position 2 of the sample rack 7a
Analysis item: One item of APTT
Reagent: the same as that in Operation Example 1.
Measurement time: until the reaction table 1 is rotated twice

Analysis Step)

(The First Cycle)

The cell transfer unit 3 supplies a new cell to the cell attachment portion 2a of P01 of the measurement port 2 at the position A.

(The Second Cycle)

50 μL of a sample sucked from the sample container housed in the sample rack 7 is dispensed to the cell in P01 at the position B. The dispensed sample is heated in a reaction unit.

(The Seventh Cycle)

At the position C, the first reagent dispensing unit 5 dispenses, to the cell in P01, 50 μL of a predetermined amount of the APTT reagent sucked from a first reagent container installed in the first reagent installation unit 9. The dispensed reagent is heated on the measurement port 2 together with the dispensed sample.

(The 26th Cycle)

At the position D, the second reagent dispensing unit 6 dispenses, to the cell in P01 of the reaction unit, 50 μL of a predetermined amount of calcium chloride liquid sucked from a second reagent container installed in the second reagent installation unit 10 to prepare a sample solution. The obtaining of measurement data (reaction measurement) is started immediately after the reagent is dispensed.

(From the 27th to 79th cycles)

The reaction measurement is continued. The reaction table 1 performs intermittent rotation operations while obtaining measurement data from the cell in P01.
The coagulation reaction progress status of APTT is analyzed in real time based on the obtained time series measurement data, and when Re is detected, the coagulation time is calculated, and the calculated coagulation time is output as the APTT value.

(The 40th Cycle)

Since it is determined that the coagulation reaction is not completed (Re is not detected) when the position E is reached, the reaction measurement is continued.

(The 80th Cycle)

Since it is determined that the coagulation reaction is completed (Re is detected) when the position E is reached, the reaction measurement of P01 is terminated, and the obtained measurement data is saved.

(The 81st Cycle)

The cell in P01 which has reached the position A is removed and discard.

<Operation Example 3> Cross Mixing Test (Delayed Reaction)

In the cross mixing test, there are an immediate reaction and a delayed reaction depending on the difference in the heating time of a sample for measurement. Typically, the heating time of the sample for measurement in the delayed reaction in the cross mixing test is 2 hours. In the present operation example, a description will be given of the rapid method of the delayed reaction in the cross mixing test in which the heating time is reduced to approximately 13 minutes.

Analysis Conditions)

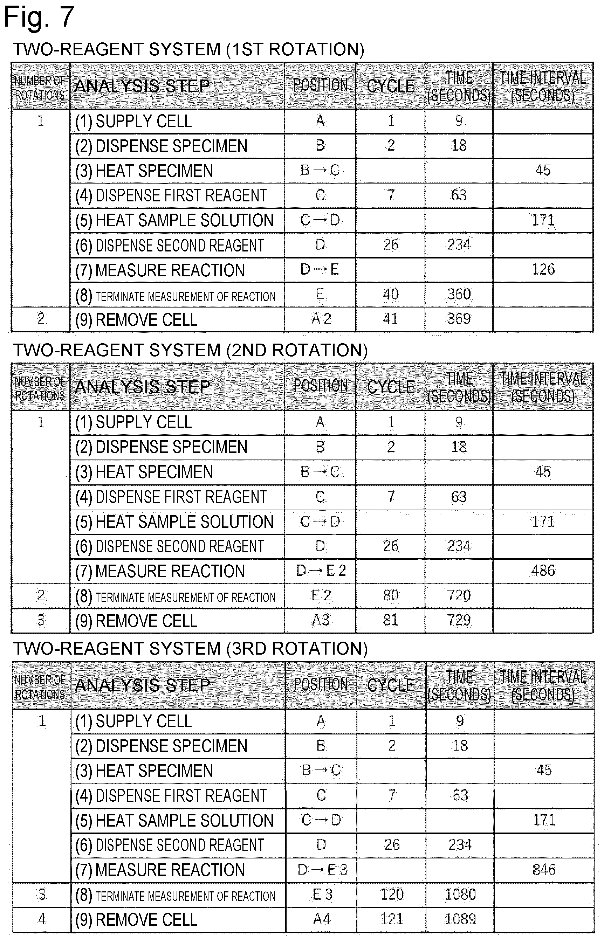

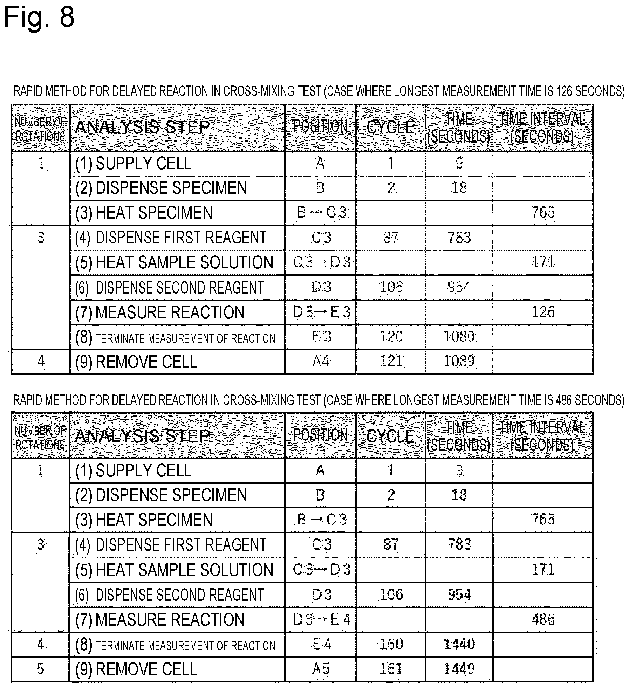

Sample: a sample at the position 3 of the sample rack 7a
Analysis item: APTT (the rapid method of the delayed reaction in the cross mixing test)
Reagent: the same as that in Operation Example 1, except for housing a normal plasma container at the position 2 of the diluent rack.
Sample: the mixing ratio of the sample and normal plasma has three conditions (each n = 1), i.e., 1:0 (sample S), 1:1 (sample M), and 0:1 (sample L).
Heating time of sample: 45 seconds for the immediate reaction, which is the same as that in the normal APTT measurement (refer to Figure 7), and 765 seconds for the delayed reaction (refer to Figure 8).
Measurement time: until the reaction table 1 is rotated three times

Analysis Step)

(The First Cycle)

S: the cell transfer unit 3 supplies a new cell to the cell attachment portion 2a of P01 of the measurement port 2 at the position A.

(The Second Cycle)

S: 50 μL of a sample sucked from a container at the position 3 of the sample rack 7 is dispensed to the cell in P01 at the position B. The dispensed sample is heated on the measurement port 2.
M: the cell transfer unit 3 supplies a new cell to the cell attachment portion 2a of P02 of the measurement port 2 at the position A.

(The Third Cycle)

M: using the sample dispensing unit 4, first, normal plasma is sucked from a container housed at the position 2 of the diluent rack 8, and then, the sample is sucked from the container at the position 3 of the sample rack 7. At the position B, 50 μL, which is obtained by combining 25 μL of the normal plasma in a probe of the dispensing unit 4 and 25 μL of the sample, is dispensed to the cell in P02. The dispensed sample and normal plasma (mixed sample) are heated on the measurement port 2.
L: the cell transfer unit 3 supplies a new cell to the cell attachment portion 2a of P03 of the measurement port 2 at the position A.

(The Fourth Cycle)

L: 50 μL of normal plasma sucked from a container at the position 2 of the diluent rack 8 is dispensed to the

14

cell in P03 at the position B. The dispensed normal plasma is heated on the measurement port 2.

(From the 5th to 86th Cycles)

Common to SML: Heating of the samples in each of the cells in P01, P02, and P03 is continued.

(The 87th Cycle)

S: at the position C of the third rotation of the reaction table 1, the first reagent dispensing unit 5 dispenses, to the cell in P01, 50 μL of a predetermined amount of the APTT reagent sucked from the first reagent container installed in the first reagent installation unit 9. After the reagent is dispensed, heating of the sample (the dispensed reagent and the heated sample) in the cell in P01 is continued.

(The 88th Cycle)

M: similar to the 87th cycle, 50 μL of the APTT reagent is dispensed to the cell in P02 at the position C. After the reagent is dispensed, heating of the sample (the dispensed reagent and the heated mixed sample) in the cell of P02 is continued.

(The 89th Cycle)

L: similar to the 87th cycle, 50 μL of the APTT reagent is dispensed to the cell of P03 at the position C. After the reagent is dispensed, heating of the sample (the dispensed reagent and the heated normal plasma) in the cell of P03 is continued.

(The 106th Cycle)

S: at the position D of the third rotation of the reaction table 1, the second reagent dispensing unit 6 dispenses, to the cell in P01, 50 μL of a predetermined amount of calcium chloride liquid sucked from the second reagent container installed in the second reagent installation unit 10 to prepare the sample solution. The obtaining of measurement data (reaction measurement) is started immediately after the reagent is dispensed.

(The 107th Cycle)

M: Similar to the 106th cycle, 50 μL of calcium chloride liquid is dispensed to the cell in P02 at the position D to prepare a sample solution. The reaction measurement is started immediately after the reagent is dispensed.

(The 108th Cycle)

L: Similar to the 106th cycle, 50 μL of calcium chloride liquid is dispensed to the cell in P03 at the position D to prepare a sample solution. The reaction measurement is started immediately after the reagent is dispensed.

(The Nth Cycle: Number of Cycles in which Second Reagent (Calcium Chloride Liquid) is Dispensed < N < Number of Cycles in which Cell is Removed)

Common to SML: for each of P01, P02, and P03, the coagulation reaction progress status is analyzed in real time based on the obtained time series measurement data, and when Re is detected, the coagulation time (APTT) is calculated, and the calculated result is output.

(The 120th Cycle)

S: since it is determined that the coagulation reaction is already completed (Re is detected) when P01 reaches the position E of the third rotation of the reaction table 1, the reaction measurement is terminated, and the obtained measurement data is saved.

(The 121st Cycle)

S: the cell in P01 which has reached the position A of the fourth rotation of the reaction table 1 is removed and discarded. M: since it is determined that the coagulation reaction is already completed (Re is detected) when P02 reaches the position E of the third rotation of the reaction table 1, the reaction measurement is terminated, and the obtained measurement data is saved.

(The 122nd Cycle)

M: the cell in P02 which has reached the position A of the fourth rotation of the reaction table 1 is removed and discarded. L: since it is determined that the coagulation reaction is already completed (Re is detected) when P03 reaches the position E of the third rotation of the reaction table, the reaction measurement is terminated, and the obtained measurement data is saved.

(The 123rd Cycle)

L: the cell in P03 which has reached the position A of the fourth rotation of the reaction table 1 is removed and discarded.

<Operation Example 4> Cross Mixing Test (Coagulation Time Extended Sample)

Analysis Conditions)

Sample: a sample at the position 3 of the sample rack 7a
Analysis item: APTT (the rapid method of the delayed reaction in the cross mixing test)
Reagent: the same as that in Operation Example 1, except for housing a normal plasma container at the position 2 of the diluent rack.
Sample: the same as that in Operation Example 3
Heating time of sample: the same as that in Operation Example 3
Measurement time: until the reaction table 1 is rotated four times (however, the measurement for the samples M and L is terminated when the reaction table 1 is rotated three times)

Analysis Step)

(From the 1st to 119th Cycles)

The same as that in Operation Example 3

(The Nth Cycle: Number of Cycles in which Second Reagent (Calcium Chloride Liquid) is Dispensed < N < Number of Cycles in which Cell is Removed)

Common to SML: for each of P01, P02, and P03, the coagulation reaction progress status is analyzed in real time based on the obtained time series measurement data, and when Re is detected, the coagulation time (APTT) is calculated, and the calculated result is output.

(The 120th Cycle)

S: since it is not determined that the coagulation reaction is completed (Re is not detected) when the position E of the third rotation of the reaction table 1 is reached, the reaction measurement is continued.

(The 121st Cycle)

M: since it is determined that the coagulation reaction is already completed (Re is detected) when P02 reaches the position E of the third rotation of the reaction table 1, the reaction measurement is terminated, and the obtained measurement data is saved.

(The 122nd Cycle)

M: the cell in P02 which has reached the position A of the fourth rotation of the reaction table 1 is removed and discarded. L: since it is determined that the coagulation reaction is already completed (Re is detected) when P03 reaches the position E of the third rotation of the reaction table 1, the reaction measurement is terminated, and the obtained measurement data is saved.

(The 123rd Cycle)

L: the cell in P03 which has reached the position A of the fourth rotation of the reaction table 1 is removed and discarded.

(The 160th Cycle)

S: since it is determined that the coagulation reaction is already completed (Re is detected) when P01 reaches the position E of the fourth rotation of the reaction table 1, the reaction measurement is terminated, and the obtained measurement data is saved.

(The 161st cycle)

S: the cell in P01 which has reached the position A of the fifth rotation of the reaction table 1 is removed and discarded.

Figures 6 to 8 illustrate tables which list how many rotations of the reaction table 1 were made, as well as an analysis step at that time, a position or interval where the analysis step was performed, a cycle in which the analysis step was performed, the time until the analysis step was started since the analysis was started, and the time required for the analysis step (specimen heating, sample solution heating, and reaction measurement) in the analysis step of Operation Examples from 1 to 4. Figure 6 illustrates a case of PT measurement, Figure 7 illustrates a case of APTT measurement, and Figure 8 illustrates a case of delayed reaction in cross mixing test. In Figure 6, an upper table is for a case where measurement was terminated when the reaction table 1 was rotated once (Operation Example 1), and a lower table is for a case where measurement was terminated when the reaction table 1 was rotated twice. In Figure 7, an upper table is for a case where measurement was terminated when the reaction table 1 was rotated once (Operation Example 1), a middle table is for a case where measurement was terminated when the reaction table 1 was rotated twice (Operation Example 2), and a lower table is for a case where measurement was terminated when the reaction table 1 was rotated three times. In Figure 8, since the delayed reaction in the cross mixing test takes time for heating a sample (a mixed solution of a subject specimen and a normal specimen), the number of times of rotation of the reaction table 1 is increased. An upper table is for a case where measurement was terminated when the reaction table 1 was rotated three times (Operation Example 3), and a lower table is for a case where measurement was terminated when the reaction table 1 was rotated four times (Operation Example 4). In the tables, the numbers given to the positions represent how many times the measurement port 2 has reached the position, and for example, E2 represents the state where the measurement port 2 has reached the position E for the second time.

As illustrated in Figures 6 and 7, in the case of the analysis module 100 illustrated in Figure 2A, while the reaction table 1 is rotated once, the measurement time of 297 seconds can be secured for the PT measurement, and 126 seconds for the APTT measurement, and while the reaction table 1 is rotated twice, the measurement time of 657 seconds can be secured for the PT measurement, and 486 seconds for the APTT measurement. Accordingly, in the cases of the PT measurement and the APTT measurement, for most samples including normal samples, the coagulation reaction is completed while the reaction table 1 is rotated once, and the samples will not enter the second rotation on the reaction table 1. Even for abnormal samples whose coagulation time is extended, in most cases, it is sufficient to measure the coagulation reaction until the reaction table 1 is rotated twice. From above, it has been found that, for most samples including normal samples and excluding samples whose coagulation time is significantly extended, from 120 to 140 seconds, which is obtained by subtracting the heating time of a specimen and a sample solution from the time for the reaction table 1 to be rotated once, is the maximum measurement time required for measurement of a coagulation reaction.

Therefore, according to the method of the present invention, while measurement can be terminated in a relatively short time for a specimen with a normal coagulation reaction and a specimen with a slight extension of coagulation time, a long measurement time can be secured for a specimen with a serious abnormality in a coagulation reaction, and with significantly extended coagulation time. Accordingly, in the method of the present invention, even when continuously analyzing a specimen group which may include abnormal specimens with extended coagulation time as well as normal specimens, it is unnecessary to set a long measurement time tailored for abnormal specimens which may exist, the throughput for analysis is not reduced.

Further, when the method of the present invention is implemented, it becomes possible to perform a cross mixing test (the rapid method for immediate reaction and delayed reaction) in parallel with usual coagulation time measurement (PT, APTT, and the like). Therefore, various kinds of blood coagulation reaction analysis can be collectively performed, without causing a significant reduction in throughput. For example, when performing continuous APTT measurement on a large number of specimens in accordance with the method of the present invention by the analysis module 100 illustrated in Figure 2A, if the analysis progresses at the same progress rate in Operation Example 1 (the reaction table 1 is rotated only once), 400 cases can be processed per hour. Here, when three cases of measurement of the delayed reaction in the cross mixing test as in Operation Example 3 is included, although the throughput per hour is temporarily reduced to 394 cases during the measurement of the three cases, when the measurement of the three cases is completed, the throughput will return to 400 cases.

Additionally, in the analysis with the method of the present invention, the reaction table 1 for measuring the cell containing the sample solution is rotated only in one direction. Therefore, swinging of the cell and shaking of the sample solution in the cell, which may occur when the table is rotated in a reciprocated manner (for example, Patent Literatures 1 and 2), do not occur. Accordingly, blurring and noise in measurement values due to such swinging and liquid permeation can be prevented.

When using the analysis module 100 illustrated in Figure 2A in the method of the present invention, the reaction table 1 is rotated only in the one direction as described above, supplying of and removing of the cell to and from the reaction table 1 are performed at one location, and the dispensing of a specimen and a reagent to the cell on the reaction table 1 is performed on a predetermined position on the reaction table 1 by controlling the operations by the rotating arms. Additionally, whether or not to terminate the measurement of the cell on the reaction table 1 and to remove the cell may be always determined at the timing when the cell on the reaction table is rotated once and is moved to a predetermined position. Therefore, the operation control of the analysis module 100 is more simple compared with a device (for example, the devices described in Patent Literatures from 1 to 3) which supplies a cell and dispenses a reagent at arbitrary positions on the reaction table 1. For example, in the devices described in Patent Literatures from 1 to 3, it is necessary to control the movement of the reaction table, the dispensing probe, and the like by recognizing the position of the measurement port for performing attachment/detachment of a cell, or dispensing of the specimen and the reagent. Additionally, when coagulation time measurements (PT, APTT, and the like) using different techniques are performed in parallel on the reaction table, the timing of the operations of the reaction table, the dispensing probe, and the like must be controlled, so that the timing of attachment/detachment of the cell, or dispensing of the specimen and the reagent do not overlap between the measurement ports. Meanwhile, in the method of the present invention, the control unit 12 of the analysis module 100 does not need to maintain or control the position of the measurement port 2 and the information on timing at which the cell should be attached/detached, or the specimen and the reagent should be dispensed. This leads to a reduction in control load of the system for blood coagulation analysis used in the method of the present invention.

Example

The coagulation reaction curves of a normal specimen and two abnormal specimens (abnormal specimens 1 and 2) with extended coagulation time were obtained by using the method of the present invention.

- In measurement of a coagulation reaction, a cell was irradiated with light having a wavelength of 660 nm using an LED as a light source, and the scattered light amount of the light scattered at 90-degree side scattered light was measured at 0.1 second intervals.
- As the coagulation reaction termination point Re, the earliest point of time at which the integration ratio $Z(i)$ at the point of time i becomes less than the threshold value Zs (1.010) was detected in accordance with the method described in WO 2021/132552. The integration ratio $Z(i)$ is the ratio of the integrated value of the coagulation reaction (scattered light amount) $R(i)$ in a minute time period, and was calculated as follows:

$$\text{integration ratio } Z(i) = Rb(i) / Ra(i)$$

$Ra(i)$ = the sum from R (i - 20) to R (i - 1)
$Rb(i)$ = the sum from R (i + 1) to R (i + 20)
$R(i)$ = the measurement data (scattered light amount) at the point of time i

- The earliest point of time at which the coagulation reaction R reached 50% or more of Re was determined as the APTT.
- The reaction measurement time (the time until a cell reached the position E from the position D) in the first rotation of the reaction table 1 was 126 seconds. When the cell is rotated twice on the reaction table 1, the reaction measurement time was 486 seconds.

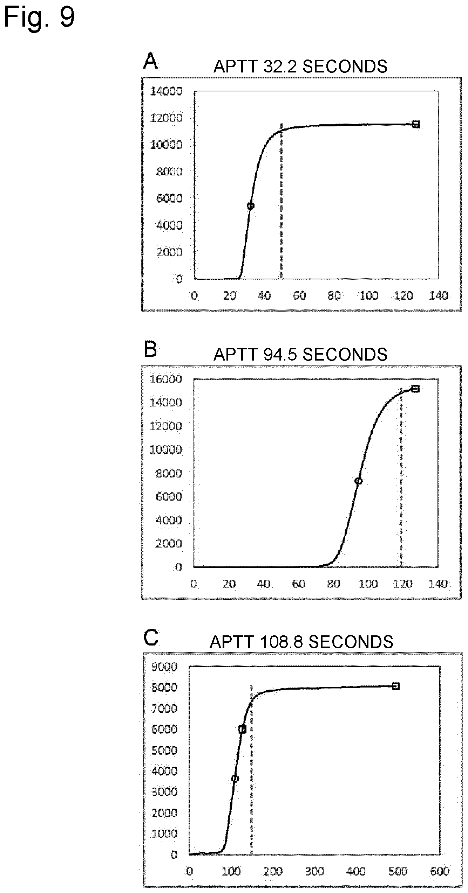

Figure 9 illustrates the coagulation reaction curves and APTTs of respective specimens. A horizontal axis in the figure represents the time, a vertical axis represents the measurement data (scattered light amount), and the coagulation reaction curves are corrected such that the data at the time when the reaction was started (at the time when the second reagent was dispensed) becomes 0. A square mark on a coagulation reaction curve represents the point at which a cell containing a specimen reached the position E on the reaction table 1, and a circle mark represents an APTT. A dotted line represents the time (tRe) at the coagulation reaction termination point Re.

Figure 9A illustrates the coagulation reaction curve of the normal specimen, Re was detected while the reaction table 1 was rotated once, and the measurement was terminated. The APTT was 32.2 seconds. Figure 9B illustrates the coagulation reaction curve of the abnormal specimen 1. Although the APTT was extended to 94.5 seconds, Re was detected while the reaction table 1 was rotated once, and the measurement was terminated. Figure 9C illustrates the coagulation reaction curve of the abnormal specimen 2. Re was not detected until the reaction table 1 was rotated once, and the measurement was terminated in the second rotation. The APTT was extended to 108.8 seconds.

Reference Signs List

1    reaction table
2    measurement port
3    cell transfer unit
4    specimen dispensing unit
5    first reagent dispensing unit
6    second reagent dispensing unit
100    analysis module

**Claims**

1. A method for analyzing a blood coagulation reaction, comprising,

   by a blood coagulation analysis device, measuring a coagulation reaction of a sample solution including a subject blood specimen;
   determining whether or not the coagulation reaction of the sample solution is completed; and
   when it is determined that the coagulation reaction is completed, terminating measurement of the coagulation reaction of the sample solution,
   wherein the blood coagulation analysis device includes a reaction table which is rotated in one direction,
   the reaction table includes a measurement port to which a cell can be attached,
   the coagulation reaction of the sample solution in the cell supplied to the measurement port is measured,
   time series data of the measured coagulation reaction is accumulated,
   whether or not the coagulation reaction is completed is determined from the accumulated time series data of the coagulation reaction, and
   when it is determined that the coagulation reaction is completed, the measurement of the coagulation reaction of the sample solution is terminated, and the cell containing the sample solution is removed from the measurement port.

2. The method according to claim 1, wherein the reaction table has a disk-like shape.

3. The method according to claim 1, wherein the reaction table performs an intermittent rotation operation, and a time for which the reaction table completes one full round of rotation is from 320 to 380 seconds.

4. The method according to claim 3, wherein an interval of the intermittent rotation operation is $\alpha$ seconds, and a number of the measurement port on the reaction table is $\beta$, where $\alpha$ = 8 to 20, $\beta$ = 16 to 47, and [$\alpha \times \beta$] = 320 to 380 seconds, and
   a rotation angle of the reaction table in one intermittent rotation operation is [$360°/\beta$].

5. The method according to any one of claims 1 to 4,
   wherein the measurement of the coagulation reaction of the sample solution including the subject blood specimen comprises:

supplying a cell to the measurement port of the reaction table;
dispensing the subject blood specimen to the cell, and heating the subject blood specimen;
dispensing a reagent to the cell containing the subject blood specimen to prepare the sample solution; and
measuring the coagulation reaction of the sample solution, and

the supplying of the cell to the measurement port, the dispensing of the subject blood specimen to the cell, and the dispensing of the reagent to the cell containing the subject blood specimen are performed when the measurement port reaches a predetermined position for each,
and
the terminating the measurement of the coagulation reaction of the sample solution is performed when the measurement port reaches a predetermined position for the terminating.

6. The method according to claim 5, wherein when the measurement port of the reaction table reaches a predetermined position A, the cell is supplied to the measurement port, when the measurement port reaches a predetermined position B, the subject blood specimen is dispensed to the cell, when the measurement port reaches a predetermined position C or a predetermined position D, the reagent is dispensed to the cell, and thereafter, when the measurement port reaches the predetermined position A or a predetermined position E, the cell is removed from the measurement port.

7. The method according to claim 6, wherein the position A is disposed on a location shifted from the position E in a rotation direction by one intermittent rotation operation of the reaction table.

8. The method according to claim 6, wherein when the cell supplied to the measurement port reaches the position E on the reaction table, if it has been determined that the coagulation reaction of the sample solution in the cell is completed, measurement of the coagulation reaction of the sample solution is terminated, and then, at the position E, or when the cell reaches the position A, the cell is removed from the measurement port.

9. The method according to claim 6, wherein when the cell supplied to the measurement port reaches the position E on the reaction table, if it has not been determined that the coagulation reaction of the sample solution in the cell is completed, the cell is rotated on the reaction table without being removed from the measurement port, and measurement of the coagulation reaction of the sample solution in the cell is continued.

10. The method according to claim 6, wherein after dispensing the subject blood specimen to the cell supplied to the measurement port at the position B, when the measurement port reaches the position C or the position D for the first time, the reagent is dispensed to the cell.

11. The method according to claim 6, wherein after dispensing the subject blood specimen to the cell at the position B, before dispensing the reagent to the cell at the position C or the position D, the reaction table is rotated one or more times, during which heating of the subject blood specimen is continued.

# Fig. 1

# Fig. 2

A

B

# Fig. 3

# Fig. 4

## A

|  |  |  |  |  |  |  |
|---|---|---|---|---|---|---|
| (1) | (2) | (3) | (4) | (5) | (6) | (7) |
| A | B |  | C |  | E | A |

SUPPLY CELL ⇒ DISPENSE SPECIMEN ⇒ HEAT SPECIMEN ⇒ DISPENSE FIRST REAGENT ⇒ MEASURE REACTION ⇒ TERMINATE MEASUREMENT OF REACTION ⇒ REMOVE CELL

## B

|  |  |  |  |  |  |  |  |  |
|---|---|---|---|---|---|---|---|---|
| (1) | (2) | (3) | (4) | (5) | (6) | (7) | (8) | (9) |
| A | B |  | C |  | D |  | E | A |

SUPPLY CELL ⇒ DISPENSE SPECIMEN ⇒ HEAT SPECIMEN ⇒ DISPENSE FIRST REAGENT ⇒ HEAT SAMPLE SOLUTION ⇒ DISPENSE SECOND REAGENT ⇒ MEASURE REACTION ⇒ TERMINATE MEASUREMENT OF REACTION ⇒ REMOVE CELL

# Fig. 5

```
        ( START MEASUREMENT )
                 │
        ┌────────────────────┐
        │    SUPPLY CELL      │ S101
        └────────────────────┘
                 │
        ┌────────────────────┐
        │ DISPENSE SPECIMEN   │ S102
        └────────────────────┘
                 │
        ┌────────────────────┐
        │  HEAT SPECIMEN      │ S103
        └────────────────────┘
                 │
        ┌────────────────────┐
        │DISPENSE FIRST REAGENT│ S104
        └────────────────────┘
                 │
                 ◇  SECOND REAGENT      S105
          No ───◇    EXISTS?
                 │  Yes
        ┌────────────────────┐
        │ HEAT MIXED SOLUTION │ S106
        └────────────────────┘
                 │
        ┌────────────────────┐
        │DISPENSE SECOND REAGENT│ S107
        └────────────────────┘
                 │
        ┌────────────────────┐
        │    MEASUREMENT      │ S108
        └────────────────────┘
                 │
                 ◇  REACTION IS         S109
                 ◇  COMPLETED?    No ──────────►
                 │  Yes                         │
                                    ┌──────────────────────┐
                                    │ CONTINUE MEASUREMENT  │ S201
                                    └──────────────────────┘
                                               │
                        ◄── Yes ──◇ REACTION IS COMPLETED?   S202
                                               │ No
                        ◄── Yes ──◇ MEASUREMENT IS           S203
                                    TERMINATED?        No ──►
                 │
        ┌────────────────────────┐
        │OUTPUT MEASUREMENT RESULT│ S110
        └────────────────────────┘
                 │
        ┌────────────────────┐
        │ TERMINATE MEASUREMENT│ S111
        └────────────────────┘
                 │
        ┌────────────────────┐
        │    REMOVE CELL      │ S112
        └────────────────────┘
                 │
        (  END MEASUREMENT  )
```

# Fig. 6

ONE-REAGENT SYSTEM (1ST ROTATION)

| NUMBER OF ROTATIONS | ANALYSIS STEP | POSITION | CYCLE | TIME (SECONDS) | TIME INTERVAL (SECONDS) |
|---|---|---|---|---|---|
| 1 | (1) SUPPLY CELL | A | 1 | 9 | |
| | (2) DISPENSE SPECIMEN | B | 2 | 18 | |
| | (3) HEAT SPECIMEN | B → C | | | 45 |
| | (4) DISPENSE FIRST REAGENT | C | 7 | 63 | |
| | (7) MEASURE REACTION | C → E | | | 297 |
| | (8) TERMINATE MEASUREMENT OF REACTION | E | 40 | 360 | |
| 2 | (9) REMOVE CELL | A 2 | 41 | 369 | |

ONE-REAGENT SYSTEM (2ND ROTATION)

| NUMBER OF ROTATIONS | ANALYSIS STEP | POSITION | CYCLE | TIME (SECONDS) | TIME INTERVAL (SECONDS) |
|---|---|---|---|---|---|
| 1 | (1) SUPPLY CELL | A | 1 | 9 | |
| | (2) DISPENSE SPECIMEN | B | 2 | 18 | |
| | (3) HEAT SPECIMEN | B → C | | | 45 |
| | (4) DISPENSE FIRST REAGENT | C | 7 | 63 | |
| | (7) MEASURE REACTION | C → E 2 | | | 657 |
| 2 | (8) TERMINATE MEASUREMENT OF REACTION | E 2 | 80 | 720 | |
| 3 | (9) REMOVE CELL | A 3 | 81 | 729 | |

# Fig. 7

TWO-REAGENT SYSTEM (1ST ROTATION)

| NUMBER OF ROTATIONS | ANALYSIS STEP | POSITION | CYCLE | TIME (SECONDS) | TIME INTERVAL (SECONDS) |
|---|---|---|---|---|---|
| 1 | (1) SUPPLY CELL | A | 1 | 9 | |
| | (2) DISPENSE SPECIMEN | B | 2 | 18 | |
| | (3) HEAT SPECIMEN | B → C | | | 45 |
| | (4) DISPENSE FIRST REAGENT | C | 7 | 63 | |
| | (5) HEAT SAMPLE SOLUTION | C → D | | | 171 |
| | (6) DISPENSE SECOND REAGENT | D | 26 | 234 | |
| | (7) MEASURE REACTION | D → E | | | 126 |
| | (8) TERMINATE MEASUREMENT OF REACTION | E | 40 | 360 | |
| 2 | (9) REMOVE CELL | A 2 | 41 | 369 | |

TWO-REAGENT SYSTEM (2ND ROTATION)

| NUMBER OF ROTATIONS | ANALYSIS STEP | POSITION | CYCLE | TIME (SECONDS) | TIME INTERVAL (SECONDS) |
|---|---|---|---|---|---|
| 1 | (1) SUPPLY CELL | A | 1 | 9 | |
| | (2) DISPENSE SPECIMEN | B | 2 | 18 | |
| | (3) HEAT SPECIMEN | B → C | | | 45 |
| | (4) DISPENSE FIRST REAGENT | C | 7 | 63 | |
| | (5) HEAT SAMPLE SOLUTION | C → D | | | 171 |
| | (6) DISPENSE SECOND REAGENT | D | 26 | 234 | |
| | (7) MEASURE REACTION | D → E 2 | | | 486 |
| 2 | (8) TERMINATE MEASUREMENT OF REACTION | E 2 | 80 | 720 | |
| 3 | (9) REMOVE CELL | A 3 | 81 | 729 | |

TWO-REAGENT SYSTEM (3RD ROTATION)

| NUMBER OF ROTATIONS | ANALYSIS STEP | POSITION | CYCLE | TIME (SECONDS) | TIME INTERVAL (SECONDS) |
|---|---|---|---|---|---|
| 1 | (1) SUPPLY CELL | A | 1 | 9 | |
| | (2) DISPENSE SPECIMEN | B | 2 | 18 | |
| | (3) HEAT SPECIMEN | B → C | | | 45 |
| | (4) DISPENSE FIRST REAGENT | C | 7 | 63 | |
| | (5) HEAT SAMPLE SOLUTION | C → D | | | 171 |
| | (6) DISPENSE SECOND REAGENT | D | 26 | 234 | |
| | (7) MEASURE REACTION | D → E 3 | | | 846 |
| 3 | (8) TERMINATE MEASUREMENT OF REACTION | E 3 | 120 | 1080 | |
| 4 | (9) REMOVE CELL | A 4 | 121 | 1089 | |

# Fig. 8

RAPID METHOD FOR DELAYED REACTION IN CROSS-MIXING TEST (CASE WHERE LONGEST MEASUREMENT TIME IS 126 SECONDS)

| NUMBER OF ROTATIONS | ANALYSIS STEP | POSITION | CYCLE | TIME (SECONDS) | TIME INTERVAL (SECONDS) |
|---|---|---|---|---|---|
| 1 | (1) SUPPLY CELL | A | 1 | 9 | |
| | (2) DISPENSE SPECIMEN | B | 2 | 18 | |
| | (3) HEAT SPECIMEN | B→C3 | | | 765 |
| 3 | (4) DISPENSE FIRST REAGENT | C3 | 87 | 783 | |
| | (5) HEAT SAMPLE SOLUTION | C3→D3 | | | 171 |
| | (6) DISPENSE SECOND REAGENT | D3 | 106 | 954 | |
| | (7) MEASURE REACTION | D3→E3 | | | 126 |
| | (8) TERMINATE MEASUREMENT OF REACTION | E3 | 120 | 1080 | |
| 4 | (9) REMOVE CELL | A4 | 121 | 1089 | |

RAPID METHOD FOR DELAYED REACTION IN CROSS-MIXING TEST (CASE WHERE LONGEST MEASUREMENT TIME IS 486 SECONDS)

| NUMBER OF ROTATIONS | ANALYSIS STEP | POSITION | CYCLE | TIME (SECONDS) | TIME INTERVAL (SECONDS) |
|---|---|---|---|---|---|
| 1 | (1) SUPPLY CELL | A | 1 | 9 | |
| | (2) DISPENSE SPECIMEN | B | 2 | 18 | |
| | (3) HEAT SPECIMEN | B→C3 | | | 765 |
| 3 | (4) DISPENSE FIRST REAGENT | C3 | 87 | 783 | |
| | (5) HEAT SAMPLE SOLUTION | C3→D3 | | | 171 |
| | (6) DISPENSE SECOND REAGENT | D3 | 106 | 954 | |
| | (7) MEASURE REACTION | D3→E4 | | | 486 |
| 4 | (8) TERMINATE MEASUREMENT OF REACTION | E4 | 160 | 1440 | |
| 5 | (9) REMOVE CELL | A5 | 161 | 1449 | |

# Fig. 9

A    APTT 32.2 SECONDS

B    APTT 94.5 SECONDS

C    APTT 108.8 SECONDS

## INTERNATIONAL SEARCH REPORT

| International application No. |
|---|
| **PCT/JP2022/036831** |

**A. CLASSIFICATION OF SUBJECT MATTER**

*G01N 35/04*(2006.01)i; *G01N 33/86*(2006.01)i
FI: G01N35/04 A; G01N33/86

According to International Patent Classification (IPC) or to both national classification and IPC

**B. FIELDS SEARCHED**

Minimum documentation searched (classification system followed by classification symbols)

G01N35/00-37/00; G01N33/86

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 8-101199 A (SHIMADZU CORPORATION) 16 April 1996 (1996-04-16) paragraphs [0010]-[0029], fig. 1-6 | 1-7, 10-11 |
| A | paragraphs [0010]-[0029], fig. 1-6 | 8-9 |
| Y | WO 2015/098473 A1 (HITACHI HIGH-TECHNOLOGIES CORP.) 02 July 2015 (2015-07-02) paragraphs [0029]-[0033], fig. 2 | 1-7, 10-11 |
| A | paragraphs [0029]-[0033], fig. 2 | 8-9 |
| Y | WO 2020/075803 A1 (LSI MEDIENCE CORP.) 16 April 2020 (2020-04-16) paragraphs [0006], [0007] | 11 |
| A | paragraphs [0006], [0007] | 1-10 |

☐ Further documents are listed in the continuation of Box C.　　☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **29 November 2022** | **13 December 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT
**Information on patent family members**

International application No.

**PCT/JP2022/036831**

| Patent document cited in search report | Publication date (day/month/year) | Patent family member(s) | Publication date (day/month/year) |
|---|---|---|---|
| JP 8-101199 A | 16 April 1996 | (Family: none) | |
| WO 2015/098473 A1 | 02 July 2015 | (Family: none) | |
| WO 2020/075803 A1 | 16 April 2020 | US 2021/0382080 A1 paragraphs [0006], [0007] EP 3865876 A1 CN 112823285 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H06109742 A
- JP H06109743 A
- JP 2000321286 A
- JP 2020068877 A
- WO 2021132552 A